# FASCICULE DE BREVET EUROPEEN

(11) **EP 1 543 778 B1**
(45) Date de publication et mention de la délivrance du brevet: **02.09.2009**
(21) Numéro de dépôt: 04292996.8
(22) Date de dépôt: 14.12.2004
(51) Int. Cl.: A61B 10/00

(54) **Flacon de préparation d'une suspension cytologique**
Fläschchen zur Herstellung einer zytologischen Suspension
Receptacle for preparing a cytological suspension

(30) Priorité: 18.12.2003 FR 0314907
(43) Date de publication de la demande: 22.06.2005
(73) Titulaire: Peltier, Eric, 92140 Clamart (FR)
(72) Inventeur: Peltier, Eric, 92140 Clamart (FR)
(74) Mandataire: Habasque, Etienne J. Jean-François

(56) Documents cités:
- EP-A- 0 520 408
- FR-A- 2 792 331
- US-A- 4 439 319
- US-A- 5 833 928
- US-A1- 2002 096 469

## Description

La présente invention concerne un flacon de préparation d'une suspension cytologique à base de fixateur.

De tels flacons sont utilisés dans l'état de la technique, pour préparer des suspensions cytologiques cervicales ou vaginales à analyser.

Les prélèvements cervicaux ou vaginaux sont effectués par des praticiens à l'aide de brosses spécifiques fixées de façon détachable sur des manches de manipulation.

Une fois le prélèvement effectué, le praticien plonge la brosse dans le flacon et détache celle-ci du manche, de manière à permettre aux cellules prélevées de se déposer dans le fixateur.

Cependant, des composants indésirables peuvent également se déposer dans le fixateur, comme par exemple, des débris récupérés par la brosse lors du prélèvement (mucus, agrégations, etc..), des squames issus du praticien et qui se déposent dans le flacon, notamment lors de la manipulation de la brosse pour la détacher du manche, etc..

Or, ces composants peuvent être très gênants lors de l'analyse ultérieure de la suspension.

Le demandeur a déjà proposé une structure particulière de flacon pour tenter de résoudre ces problèmes.

Ce flacon est décrit dans le document FR-A-2 792 331.

Le préambule de la revendication 1 est basé sur ce document.

Selon ce document, ce flacon est muni d'une ouverture destinée à recevoir une brosse de prélèvement cytologique, fixée de façon détachable sur un manche de manipulation, et est caractérisé en ce que l'ouverture du flacon comporte des moyens de butée pour la brosse, permettant de bloquer celle-ci dans le flacon et de la détacher du manche et au moins une portion de voile ajourée de filtrage de la suspension lors d'un versement.

Cependant, à l'usage, un tel flacon a également présenté un certain nombre d'inconvénients notamment au niveau du colmatage de la portion de voile ajourée de filtrage de la suspension par les composants mentionnés précédemment.

Le but de l'invention est donc de perfectionner encore ces flacons de préparation.

A cet effet, l'invention a pour objet un flacon de préparation d'une suspension cytologique à base de fixateur, muni d'une ouverture destinée à recevoir une brosse de prélèvement cytologique fixée de façon détachable sur un manche de manipulation, caractérisé en ce qu'il comporte un panier filtrant de réception de la brosse, au moins en partie immergé dans la suspension.

Suivant d'autres caractéristiques :
- le panier filtrant est fixé de façon détachable dans le flacon pour permettre son retrait et donc celui de la brosse et récupérer la suspension ;
- le panier est vissé dans le flacon ;
- le panier et le flacon comprennent des moyens d'accrochage élastiques ;
- le fond du flacon est muni de moyens de vidange de la suspension ;
- le flacon comporte deux parties fixées l'une sur l'autre de façon détachable, dont la partie supérieure porte le panier filtrant et dont la partie inférieure contient la suspension pour permettre sa récupération ;
- les deux parties du flacon comprennent des moyens de vissage complémentaires ;
- une partie du panier filtrant est située hors de la suspension et comporte un trou de récupération de la suspension par versement ou aspiration.

L'invention sera mieux comprise à la lecture de la description qui va suivre, donnée uniquement à titre d'exemple et faite en se référant aux dessins annexés, sur lesquels :
- les Figs.1 et 2 représentent de vues en coupe schématiques d'un premier exemple de réalisation d'un flacon selon l'invention ;
- les Figs.3 et 4 représentent des vues en coupe schématiques d'un deuxième exemple de réalisation d'un flacon selon l'invention ; et
- les Figs.5 et 6 représentent des vues en coupe schématiques respectivement d'un troisième et d'un quatrième exemples de réalisation d'un flacon selon l'invention.

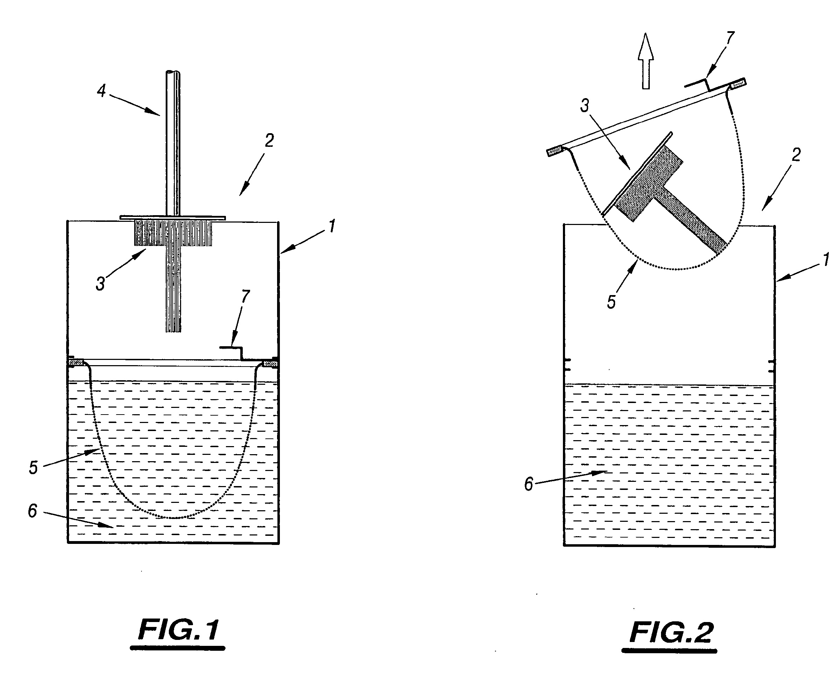

On a en effet illustré sur les figures 1 et 2, un premier exemple de réalisation d'un flacon selon l'invention.

Ce flacon est un flacon de préparation d'une suspension cytologique à base de fixateur.

Ce flacon est désigné par la référence générale 1 et comporte donc une ouverture désignée par la référence générale 2, permettant le passage d'une brosse de prélèvement cytologique désignée par la référence générale 3, fixée de façon détachable sur un manche de manipulation désigné par la référence générale 4.

Selon l'invention, ce flacon comporte un panier filtrant de réception de la brosse, au moins en partie immergé dans la suspension.

Ce panier réalisé en tout matériau approprié de filtrage, est désigné par la référence générale 5 sur ces figures 1 et 2 et la suspension est désignée par la référence générale 6.

Par ailleurs, le panier filtrant peut également être muni de moyens de butée 7 formés par exemple par une partie en saillie en forme de crochet ou autres de celui-ci, permettant de détacher la brosse du manche et de faire tomber cette brosse dans le panier et donc dans la suspension.

Dans l'exemple de réalisation illustré sur les figures 1 et 2, le panier filtrant 5 est fixé de façon détachable dans le flacon pour permettre son retrait et donc celui de la brosse et récupérer la suspension.

Ainsi par exemple, le panier peut être vissé dans le flacon ou ce panier et ce flacon peuvent comprendre des moyens d'accrochage élastiques, permettant de retirer le panier et donc la brosse du flacon, comme cela est illustré sur la figure 2.

Tous les composants indésirables sont alors retenus par le panier filtrant, de sorte qu'après retrait de celui-ci, la suspension peut être récupérée.

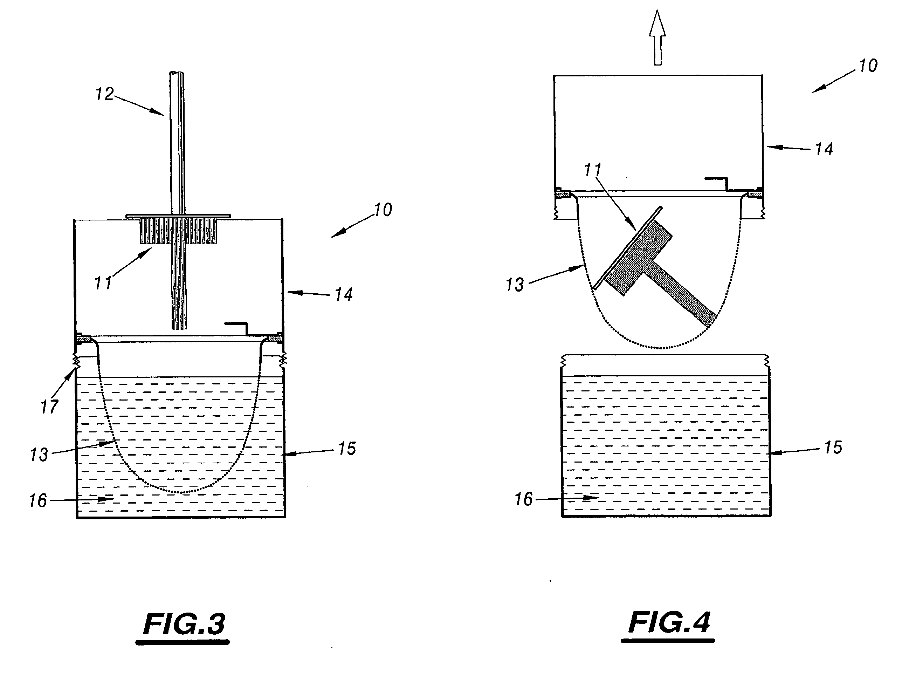

Bien entendu, d'autres modes de réalisation peuvent être envisagés, comme celui illustré sur les figures 3 et 4.

Sur ces figures, le flacon est désigné par la référence générale 10, la brosse par la référence générale 11, le manche par la référence générale 12 et le panier filtrant de réception de la brosse, par la référence générale 13.

Dans cet exemple de réalisation, le flacon comporte deux parties fixées l'une sur l'autre de façon détachable, désignées par les références générales 14 et 15 respectivement.

Le panier 13 est alors relié à la partie supérieure 14 du flacon, tandis que la suspension 16 est contenue dans la partie inférieure de ce flacon désignée par la référence générale 15.

Ces deux parties de flacon sont fixées de manière détachable l'une sur l'autre, par exemple par l'intermédiaire de moyens de vissage complémentaires désignés par la référence générale 17, prévus dans la portion supérieure de la partie inférieure 15 du flacon et dans la portion inférieure de la partie supérieure 14 de ce flacon.

La récupération de la suspension se fait alors par dévissage de la partie inférieure 15 du flacon comme cela est illustré sur la figure 4.

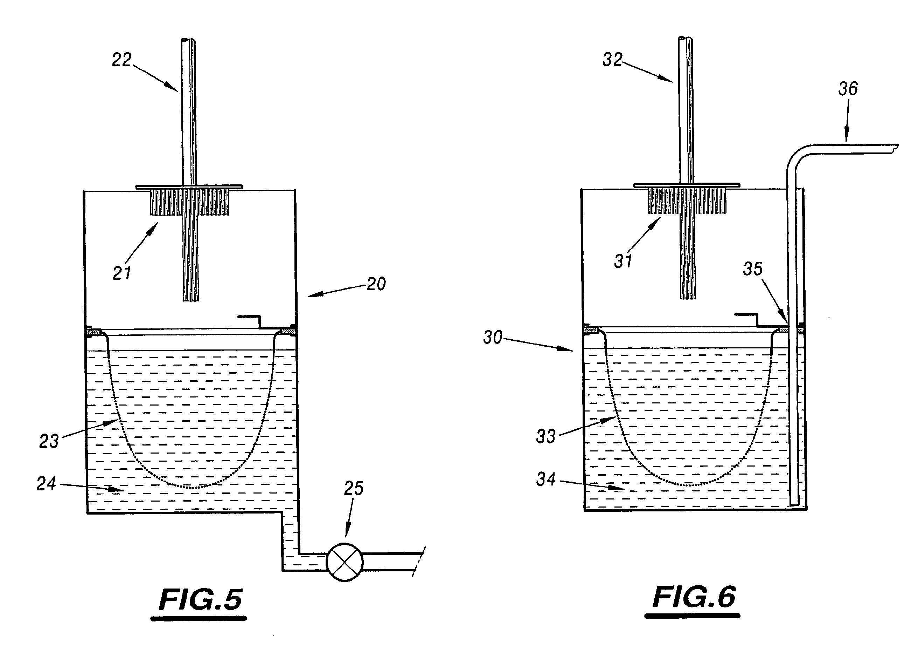

Sur la figure 5, on a illustré un autre exemple de réalisation où l'on reconnaît un flacon 20, une brosse 21, un manche 22, un panier filtrant 23 et une suspension 24.

Dans cet exemple de réalisation, le fond du flacon est muni de moyens de vidange de la suspension désignés par la référence générale 25 et formés par exemple par des moyens formant vanne, actionnables par un utilisateur pour récupérer la suspension par écoulement.

Enfin sur la figure 6, on a encore illustré un autre exemple de réalisation dans lequel le flacon est désigné par la référence générale 30, la brosse par la référence 31, le manche par la référence 32, le panier filtrant par la référence 33 et la suspension par la référence 34.

Dans cet exemple de réalisation, une partie du panier filtrant 33 est située hors de la suspension 34 et comporte un trou de récupération de la suspension par versement ou aspiration.

Ce trou est par exemple désigné par la référence générale 35 et permet le passage d'un conduit d'aspiration ou de versement par exemple 36, permettant de récupérer la suspension 34.

Bien entendu, d'autres modes de réalisation tombant sous l'étendue de la protection définie par les revendications suivantes. peuvent être envisagés.

## Revendications

1. Flacon de préparation d'une suspension cytologique à base de fixateur, muni d'une ouverture (2) destinée à recevoir une brosse de prélèvement cytologique (3 ; 11 ; 21 ; 31) fixée de façon détachable sur un manche de manipulation (4 ; 12 ; 22 ; 32), **caractérisé en ce qu'**il comporte un panier filtrant (5 ; 13 ; 23 ; 33) de réception de la brosse, au moins en partie immergé dans la suspension (6 ; 16 ; 24 ; 34).

2. Flacon selon la revendication 1, **caractérisé en ce que** le panier filtrant (5) est fixé de façon détachable dans le flacon (1) pour permettre son retrait et donc celui de la brosse et récupérer la suspension.

3. Flacon selon la revendication 2, **caractérisé en ce que** le panier (5) est vissé dans le flacon (1).

4. Flacon selon la revendication 2, **caractérisé en ce que** le panier (5) et le flacon (1) comprennent des moyens d'accrochage élastiques.

5. Flacon selon la revendication 1, **caractérisé en ce que** le fond du flacon (20) est muni de moyens de vidange (25) de la suspension (24).

6. Flacon selon la revendication 1, **caractérisé en ce que** le flacon (10) comporte deux parties (14,15) fixées l'une sur l'autre de façon détachable, dont la partie supérieure (14) porte le panier filtrant (13) et dont la partie inférieure (15) contient la suspension (16) pour permettre sa récupération.

7. Flacon selon la revendication 6, **caractérisé en ce que** les deux parties (14,15) du flacon comprennent des moyens de vissage complémentaires (17).

8. Flacon selon la revendication 1, **caractérisé en ce qu'**une partie du panier filtrant (33) est située hors de la suspension (34) et comporte un trou (35) de récupération de la suspension (34) par versement ou aspiration.

9. Flacon selon l'une quelconque des revendications 1 à 8, **caractérisé en ce que** le panier filtrant (5 ;13 ; 23 ; 33) est muni de moyens de butée (7) permettant de détacher la brosse du manche et de faire tomber cette brosse dans le panier filtrant.

10. Flacon selon la revendication 9, **caractérisé en ce que** lesdits moyens de butée (7) sont formés par une partie en saillie en forme de crochet.

## Claims

1. Bottle for preparing a fixative-based cytological suspension, having an opening (2) adapted to receive a brush for taking a cytological sample (3; 11; 21; 31) detachably fixed to a manipulating handle (4; 12; 22; 32), **characterised in that** it comprises a filter basket (5; 13; 23; 33) for accommodating the brush, this basket being at least partly immersed in the suspension (6; 16; 24; 34).

2. Bottle according to claim 1, **characterised in that** the filter basket (5) is detachably fixed in the bottle (1) to enable it and hence the brush to be removed and the suspension recovered.

3. Bottle according to claim 2, **characterised in that** the basket (5) is screwed into the bottle (1).

4. Bottle according to claim 2, **characterised in that** the basket (5) and the bottle (1) comprise resilient attachment means.

5. Bottle according to claim 1, **characterised in that** the bottom of the bottle (20) is provided with means (25) for draining off the suspension (24).

6. Bottle according to claim 1, **characterised in that** the bottle (10) comprises two parts (14, 15) detachably fixed one on the other, the upper part (14) carrying the filter basket (13) and the lower part (15) containing the suspension (16) to enable it to be recovered.

7. Bottle according to claim 6, **characterised in that** the two parts (14, 15) of the bottle comprise complementary screwing means (17).

8. Bottle according to claim 1, **characterised in that** part of the filter basket (33) is located outside the suspension (34) and has a hole (35) for recovering the suspension (34) by pouring or aspiration.

9. Bottle according to any one of claims 1 to 8, **characterised in that** the filter basket (5; 13; 23; 33) is provided with abutment means (7) enabling the brush to be detached from the handle and this brush to be dropped into the filter basket.

10. Bottle according to claim 9, **characterised in that** the abutment means (7) are formed by a projecting part in the shape of a hook.

## Patentansprüche

1. Fläschchen zur Herstellung einer zytologischen Suspension auf Fixiermittelbasis, versehen mit einer Öffnung (2), bestimmt zur Aufnahme einer zytologischen Abstrichbürste (3; 11; 21; 31), abnehmbar befestigt an einem Handhabungsgriff (4; 12; 22; 32),
**dadurch gekennzeichnet, dass** es zur Aufnahme der Bürste einen wenigstens teilweise in die Suspension (6; 16; 24; 34) eingetauchten Filterkorb (5; 13; 23; 33) umfasst.

2. Fläschchen nach Anspruch 1, **dadurch gekennzeichnet, dass** der Filterkorb (5) abnehmbar in dem Fläschchen (1) befestigt ist, um seine Herausnahme und folglich die der Bürste zu ermöglichen und die Suspension zurückzugewinnen.

3. Fläschchen nach Anspruch 2, **dadurch gekennzeichnet, dass** der Korb (5) in das Fläschchen (1) hineingeschraubt ist.

4. Fläschchen nach Anspruch 2, **dadurch gekennzeichnet, dass** der Korb (5) und das Fläschchen (1) elastische Befestigungseinrichtungen umfassen.

5. Fläschchen nach Anspruch 1, **dadurch gekennzeichnet, dass** der Boden des Fläschchens (20) Entleerungseinrichtungen (25) der Suspension (24) umfasst.

6. Fläschchen nach Anspruch 1, **dadurch gekennzeichnet, dass** das Fläschchen (10), um die Zurückgewinnung der Suspension zu ermöglichen, zwei lösbar aufeinander befestigte Teile (14, 15) umfasst, von denen der obere Teil (14) den Filterkorb (13) trägt und der untere Teil (15) die Suspension (16) enthält.

7. Fläschchen nach Anspruch 6, **dadurch gekennzeichnet, dass** die beiden Teile (14, 15) des Fläschchens komplementäre Verschraubungseinrichtungen (17) umfassen.

8. Fläschchen nach Anspruch 1, **dadurch gekennzeichnet, dass** ein Teil des Filterkorbs (33) sich außerhalb der Suspension befindet und ein Loch (35) zur Zurückgewinnung der Suspension (34) durch Gießen oder Saugen umfasst.

9. Fläschchen nach einem der Ansprüche 1 bis 8, **dadurch gekennzeichnet, dass** der Filterkorb (5; 13; 23; 33) Anschlageinrichtungen (7) umfasst, die ermöglichen, die Bürste von dem Griff zu lösen und diese Bürste in den Filterkorb fallen zu lassen.

10. Fläschchen nach Anspruch 1, **dadurch gekennzeichnet, dass** die genannten Anschlageinrichtungen (7) durch einen hackenförmig vorstehenden Teil gebildet werden.
